(19) Europäisches Patentamt
European Patent Office
Office européen des brevets

(11) EP 0 876 340 B1

(12) **FASCICULE DE BREVET EUROPEEN**

(45) Date de publication et mention de la délivrance du brevet:
**20.03.2002 Bulletin 2002/12**

(21) Numéro de dépôt: **96938280.3**

(22) Date de dépôt: **08.11.1996**

(51) Int Cl.[7]: **C07C 323/22**, C07C 319/20, C07B 41/06

(86) Numéro de dépôt international:
**PCT/FR96/01763**

(87) Numéro de publication internationale:
**WO 97/17324 (15.05.1997 Gazette 1997/21)**

(54) **PROCEDE D'ACYLATION D'UN THIOETHER AROMATIQUE**

VERFAHREN ZUR ACYLIERUNG VON AROMATISCHEN THIOETHERN

AROMATIC THIOETHER ACYLATION METHOD

(84) Etats contractants désignés:
**AT BE CH DE DK ES FI FR GB GR IE IT LI LU MC NL PT SE**

(30) Priorité: **10.11.1995 FR 9513310**

(43) Date de publication de la demande:
**11.11.1998 Bulletin 1998/46**

(73) Titulaire: **RHONE-POULENC CHIMIE**
**92408 Courbevoie Cédex (FR)**

(72) Inventeurs:
- **GILBERT, Laurent**
  **F-69007 Lyon (FR)**
- **SPAGNOL, Michel**
  **F-69003 Lyon (FR)**

(74) Mandataire: **Dutruc-Rosset, Marie-Claude**
**Rhodia Services,**
**Direction de la Propriété Industrielle,**
**40, rue de la Haie-Coq**
**93306 Aubervilliers Cedex (FR)**

(56) Documents cités:
**EP-A- 0 334 096** **EP-A- 0 459 495**
**FR-A- 2 070 102** **FR-A- 2 370 472**
**FR-A- 2 667 063** **US-A- 4 593 125**

- **J. CHEM. SOC., CHEM. COMMUN., 1993, pages 1157-1158, XP002004929 A. KAWADA ET AL:**
- **STUD. SURF. SCI. CATAL. (HETEROG. CATAL. FINE CHEM.), vol. 41, 1988, pages 241-248, XP002004930 B. COQ ET AL:**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 876 340 B1

Printed by Jouve, 75001 PARIS (FR)

## Description

La présente invention a pour objet un procédé d'acylation d'un thioéther aromatique.

Dans sa variante préférée, l'invention réside dans un procédé de condensation de l'anhydride acétique ou du chlorure d'acétyle avec le thioanisole.

Dans l'exposé qui suit de la présente invention, on entend "par thioéther aromatique", un composé aromatique dont un atome d'hydrogène directement lié au noyau aromatique est remplacé par un groupe thioéther et par "composé aromatique", la notion classique d'aromaticité telle que définie dans la littérature, notamment par Jerry MARCH, Advanced Organic Chemistry, 4ème édition, John Wiley and Sons, 1992, pp. 40 et suivantes.

Il est connu selon FR 2 667 063, d'effectuer la benzoylation d'un composé aromatique, en particulier d'un thioéther aromatique, par réaction de ce dernier avec un acide carboxylique aromatique, en présence d'une zéolither de type HY ou Hβ.

Il a maintenant été trouvé et c'est ce qui constitue l'objet de la présente invention, un procédé d'acylation d'un thioéther aromatique caractérisé par le fait qu'il consiste à faire réagir ledit thioéther avec un agent d'acylation choisi dans le groupe formé par les halogénures d'acides carboxyliques aliphatiques ou cycloaliphatiques et les anhydrides d'acides carboxyliques aliphatiques ou cycloaliphatiques, en présence d'une quantité efficace d'une zéolithe acide.

Plus précisément, la présente invention a pour objet un procédé d'acylation d'un thioéther aromatique de formule générale (I) :

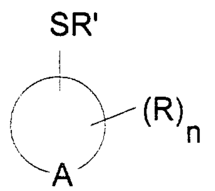

(I)

dans laquelle :

- A symbolise le reste d'un cycle formant tout ou partie d'un système carbocyclique aromatique, monocyclique ou polycyclique, système comprenant au moins un groupe SR' : ledit reste cyclique pouvant porter un ou plusieurs substituants,
- le ou les radicaux R, identiques ou différents, représentent l'un des atomes ou groupes suivants :
  . un atome d'hydrogène,
  . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
  . un radical alcényle linéaire ou ramifié ayant de 2 à 6 atomes de carbone, de préférence, de 2 à 4 atomes de carbone, tel que vinyle, allyle,
  . un radical cyclohexyle ou benzyle,
  . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy, isobutoxy, sec-butoxy, tert-butoxy,
  . un groupe acyle ayant de 2 à 6 atomes de carbone,
  . un groupe hydroxyle,
  . un atome d'halogène, de préférence un atome de fluor, chlore ou brome, un radical trifluorométhyle,
  . un groupe amine,
- R' représente un radical hydrocarboné éventuellement substitué ayant de 1 à 24 atomes de carbone, qui peut être un radical aliphatique acyclique saturé ou insaturé, linéaire ou ramifié ; un radical cycloaliphatique saturé, insaturé ou aromatique, monocyclique ou polycyclique ; un radical aliphatique saturé ou insaturé, linéaire ou ramifié, porteur d'un substituant cyclique,
- R' et R peuvent former un cycle comprenant éventuellement un autre hétéroatome,
- n représentant le nombre de substituants sur un noyau aromatique est un nombre inférieur ou égal à 4,

Dans le présent texte, on désigne, de manière simplifiée, par "groupes thioéther", les groupes du type -S-R' dans lesquels R' a la signification donnée précédemment. R' représente donc aussi bien un radical aliphatique acyclique ou cycloaliphatique, saturé, insaturé ou aromatique qu'un radical aliphatique saturé ou insaturé porteur d'un substituant

cyclique.

Le thioéther aromatique qui intervient dans le procédé de l'invention répond à la formule (I) dans laquelle R' représente un radical aliphatique acyclique, saturé ou insaturé, linéaire ou ramifié.

Plus préférentiellement, R' représente un radical alkyle linéaire ou ramifié ayant de 1 à 12 atomes de carbone, de préférence de 1 à 6 atomes de carbone : la chaîne hydrocarbonée pouvant être éventuellement interrompue par un hétéroatome (par exemple, l'oxygène), par un groupe fonctionnel (par exemple -CO-) et/ou porteuse d'un substituant (par exemple, un halogène).

Le radical aliphatique acyclique, saturé ou insaturé, linéaire ou ramifié peut être éventuellement porteur d'un substituant cyclique. Par cycle, on entend de préférence, un cycle carbocyclique saturé, insaturé ou aromatique, de préférence cycloaliphatique ou aromatique notamment cycloaliphatique comprenant 6 atomes de carbone dans le cycle ou benzénique.

Le radical aliphatique acyclique peut être relié au cycle par un lien valentiel, un hétéroatome ou un groupe fonctionnel et des exemples sont donnés ci-dessus.

Le cycle peut être éventuellement substitué et à titre d'exemples de substituants cycliques, on peut envisager, entre autres, les substituants tels que R dont la signification est précisée pour la formule (Ia).

R' peut représenter également un radical carbocyclique saturé ou comprenant 1 ou 2 insaturations dans le cycle, ayant généralement de 3 à 8 atomes de carbone, de préférence, 6 atomes de carbone dans le cycle ; ledit cycle pouvant être substitué avec des substituants tels que R.

R' peut représenter également un radical carbocyclique aromatique, de préférence monocyclique ayant généralement au moins 4 atomes de carbone, de préférence, 6 atomes de carbone dans le cycle ; ledit cycle pouvant être substitué avec des substituants tels que R.

Le procédé de l'invention s'applique tout particulièrement aux thioéthers aromatiques de formule (I) dans laquelle R' représente un radical alkyle linéaire ou ramifié ayant de 1 à 4 atomes de carbone ou un radical phényle.

Comme exemples de radicaux R' préférés selon l'invention, on peut citer les radicaux méthyle et éthyle.

Dans la formule générale (I) des thioéthers aromatiques, le reste A peut représenter le reste d'un composé carbocyclique aromatique, monocyclique ayant au moins 4 atomes de carbone et de préférence 6 atomes de carbone ou le reste d'un composé carbocyclique polycyclique qui peut être constitué par au moins 2 carbocycles aromatiques et formant entre eux des systèmes ortho- ou ortho- et péricondensés ou par au moins 2 carbocycles dont au moins l'un d'entre eux est aromatique et formant entre eux des systèmes ortho- ou ortho- et péricondensés. On peut citer plus particulièrement, un reste naphtalénique.

Le reste A peut porter un ou plusieurs substituants sur le noyau aromatique.

Le nombre de substituants présents sur le cycle dépend de la condensation en carbone du cycle et de la présence ou non d'insaturations sur le cycle.

Le nombre maximum de substituants susceptibles d'être portés par un cycle, est aisément déterminé par l'Homme du Métier.

Dans le présent texte, on entend par "plusieurs", généralement, moins de 4 substituants sur un noyau aromatique. Des exemples de substituants sont donnés ci-dessous mais cette liste ne présente pas de caractère limitatif. N'importe quel substituant peut être présent sur le cycle dans la mesure où il n'interfère pas au niveau du produit désiré.

Le procédé de l'invention s'applique plus particulièrement, aux thioéthers aromatiques de formule (Ia):

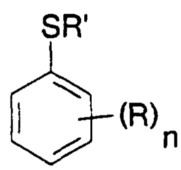

(Ia)

dans laquelle :

- n est un nombre inférieur ou égal à 4, de préférence égal à 0, 1 ou 2,
- le radical R' représente un radical alkyle, linéaire ou ramifié, ayant de 1 à 6 atomes de carbone, de préférence de 1 à 4 atomes de carbone, éventuellement interrompu par un atome d'oxygène ou un groupe carbonyle et/ou porteur d'un ou plusieurs atomes d'halogène, de préférence, un atome de chlore, ou un radical phényle,
- le ou les radicaux R représentent l'un des atomes ou groupes suivants :
  - . un atome d'hydrogène,
  - . un radical alkyle, linéaire ou ramifié, ayant de 1 à 6 atomes de carbone, de préférence de 1 à 4 atomes de

carbone, tel que méthyle, éthyle, propyle, isopropyle, butyle, isobutyle, sec-butyle, tert-butyle,

. un radical alcényle linéaire ou ramifié ayant de 2 à 6 atomes de carbone, de préférence, de 2 à 4 atomes de carbone, tel que vinyle, allyle,
. un radical cyclohexyle ou benzyle,
. un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy, isobutoxy, sec-butoxy, tert-butoxy,
. un groupe acyle ayant de 2 à 6 atomes de carbone,
. un groupe hydroxyle,
. un atome d'halogène, de préférence un atome de fluor, chlore ou brome, un radical trifluorométhyle,
. un groupe amine,

- deux groupes R placés sur deux atomes de carbone vicinaux peuvent former ensemble et avec les atomes de carbone qui les portent un cycle benzénique,
- les radicaux SR' et R et les 2 atomes successifs du cycle benzénique peuvent former entre eux, un cycle ayant de 5 à 7 atomes, comprenant éventuellement un autre hétéroatome.

Lorsque n est supérieur ou égal à 1, les radicaux R' et R et les 2 atomes successifs du cycle benzénique peuvent être liés entre eux par un radical alkylène, alcénylène ou alcénylidène ayant de 2 à 4 atomes de carbone pour former un hétérocycle saturé, insaturé ou aromatique ayant de 5 à 7 atomes de carbone. Un ou plusieurs atomes de carbone peuvent être remplacés par un autre hétéroatome, de préférence le soufre. Ainsi, les radicaux SR' et R peuvent représenter un radical méthylène dithio ou éthylène dithio.

Le procédé de l'invention s'applique plus particulièrement aux thioéthers aromatiques de formule (Ia) dans laquelle n est égal à 1, le radical R' représente un radical alkyle ayant de 1 à 4 atomes de carbone et R représente un atome d'hydrogène, un radical alkyle ou alkoxy ayant de 1 à 4 atomes de carbone ou un groupe hydroxyle.

A titre illustratif de composés répondant à la formule (I), on peut mentionner plus particulièrement :

- le thioanisole,
- l'o-thiocrésol,
- le m-thiocrésol,
- le p-thiocrésol,
- le 2-thioéthylnaphtalène,
- le S-phénylthioacétate,
- le 3-(méthylmercapto)aniline,
- le S-phénylthiopropionate.

Le composé auquel s'applique de manière plus particulièrement intéressante le procédé selon l'invention est le thioanisole.

Il est souhaitable de mettre en oeuvre un thioéther aromatique présentant une bonne pureté chimique. Une pureté d'au moins 97 % est souhaitable.

Il peut s'avérer nécessaire de faire une purification du substrat de départ, par exemple par distillation dans la mesure où il contient des impuretés susceptibles d'empoisonner le catalyseur zéolithique.

Pour ce qui est du réactif d'acylation, il est choisi dans le groupe formé par les halogénures d'acides carboxyliques et les anhydrides d'acides carboxyliques.

Lesdits dérivés proviennent de préférence, des acides carboxyliques aliphatiques, saturés ou insaturés, linéaires ou ramifiés ou des acides cycloaliphatiques, éventuellement substitués, saturés ou insaturés.

Ils répondent plus particulièrement à la formule (II) :

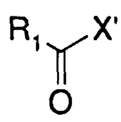
(II)

dans laquelle :

- $R_1$ représente :

. un radical aliphatique saturé ou insaturé, linéaire ou ramifié ayant de 1 à 24 atomes de carbone ; un radical

cycloaliphatique, saturé ou insaturé, monocyclique ou polycyclique, ayant de 3 à 12 atomes de carbone ;

- X' représente :
  - . un atome d'halogène, de préférence un atome de chlore ou de brome,
  - . un radical $-O-CO-R_2$ avec $R_2$, identique ou différent de $R_1$, ayant la même signification que $R_1$ : $R_1$ et $R_2$ pouvant former ensemble un radical divalent aliphatique saturé ou insaturé, linéaire ou ramifié, ayant au moins 2 atomes de carbone.

Par substituant cyclique, on se réfère à ce qui est décrit précédemment.
Plus préférentiellement, $R_1$ représente un radical alkyle linéaire ou ramifié ayant de 1 à 12 atomes de carbone, de préférence de 1 à 6 atomes de carbone : la chaîne hydrocarbonée pouvant être éventuellement interrompue par un hétéroatome (par exemple, l'oxygène), par un groupe fonctionnel (par exemple -CO-) et/ou porteuse d'un substituant (par exemple, un halogène ou un groupe $CF_3$).
$R_1$ représente de préférence un radical alkyle ayant de 1 à 4 atomes de carbone, tel que méthyle, éthyle, propyle, isopropyle, butyle, isobutyle, sec-butyle, tert-butyle.
$R_1$ représente également un radical alcényle ayant de 2 à 10 atomes de carbone, tel que vinyle, propèn-yle, butèn-yle, pentèn-yle, hexèn-yle, octèn-yle, décèn-yle.
Le radical $R_1$ représente également un radical non aromatique, de préférence, cycloaliphatique, par exemple, un radical cyclohexyle, qui peut être éventuellement substitué. N'importe quel substituant peut être présent sur le cycle dans la mesure où il n'interfère pas au niveau du produit souhaité.
Comme exemples plus particuliers de substituants, on peut citer, notamment :

- . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
- . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy, isobutoxy, sec-butoxy, tert-butoxy,
- . un atome d'halogène, de préférence un atome de fluor, chlore ou brome.

Les agents d'acylation préférés sont les anhydrides d'acides. Ils répondent plus particulièrement à la formule (II) dans laquelle $R_1$ et $R_2$ sont identiques et représentent un radical alkyle ayant de 1 à 4 atomes de carbone, éventuellement porteurs d'atomes d'halogène, de préférence, de chlore.
Lorsque l'agent d'acylation est un halogénure d'acide, il répond préférentiellement à la formule (II) dans laquelle X' représente un atome de chlore et $R_1$ représente un radical alkyle ayant de 1 à 4 atomes de carbone, de préférence, méthyle ou éthyle éventuellement porteur d'atomes d'halogène, de préférence, de chlore.
A titre illustratif d'agents d'acylation répondant à la formule (II), on peut citer plus particulièrement :

- l'anhydride acétique,
- l'anhydride propanoïque,
- l'anhydride isobutyrique,
- l'anhydride trifluoroacétique,
- l'anhydride trichloroacétique,
- l'anhydride de monochloracétyle,
- l'anhydride de dichloroacétyle,
- le chlorure d'acétyle,
- le chlorure de monochloracétyle,
- le chlorure de dichloroacétyle,
- le chlorure de propanoyle,
- le chlorure d'isobutanoyle,
- le chlorure de pivaloyle,
- le chlorure de crotonyle.

Conformément au procédé de l'invention, on effectue la réaction d'acylation en présence d'un catalyseur constitué par une zéolithe acide.
Par "zéolithe", on entend un tectosilicate cristallisé d'origine naturelle ou synthétique dont les cristaux résultent de l'assemblage tridimensionnel d'unités tétraédriques de $SiO_4$ et $TO_4$ : T représentant un élément trivalent tel que aluminium, gallium, bore, fer, de préférence, l'aluminium.
Les zéolithes de type aluminosilicate sont les plus communes.

Les zéolithes présentent au sein du réseau cristallin, un système de cavités reliées entre elles par des canaux d'un diamètre bien défini que l'on appelle les pores.

Les zéolithes peuvent présenter un réseau de canaux monodimensionnel, bidimensionnel ou tridimensionnel.

Dans le procédé de l'invention, on peut faire appel à une zéolithe naturelle ou synthétique.

Comme exemples de zéolithes naturelles susceptibles d'être utilisées, on peut citer, par exemple : la chabazite, la clinoptilolite, l'érionite, la phillipsite, l'offrétite.

Conviennent tout à fait bien à la mise en oeuvre de l'invention, les zéolithes synthétiques.

Comme exemples de zéolithes synthétiques à réseau monodimensionnel, on peut citer entre autres, la zéolithe ZSM-4, la zéolithe L, la zéolithe ZSM-12, la zéolithe ZSM-22, la zéolithe ZSM-23, la zéolithe ZSM-48.

A titre d'exemples de zéolithes à réseau bidimensionnel mises en oeuvre préférentiellement, on peut mentionner la mordénite, la ferrierite.

En ce qui concerne les zéolithes à réseau tridimensionnel, on peut nommer plus particulièrement, la zéolithe β, la zéolithe Y, la zéolithe X, la zéolithe ZSM-5, la zéolithe ZSM-11, l'offrétite.

On fait appel préférentiellement aux zéolithes synthétiques et plus particulièrement aux zéolithes qui sont sous les formes suivantes :

- la mazzite de rapport molaire Si/Al de 3,4,
- la zéolithe L de rapport molaire Si/Al de 1,5 à 3,5,
- la mordénite de rapport molaire Si/Al de 5 à 150, de préférence, de 10 à 100 et encore plus préférentiellement de 10 à 25,
- la ferrierite de rapport molaire Si/Al de 3 à 10,
- l'offrétite de rapport molaire Si/Al de 4 à 8,5.
- les zéolithes β de rapport molaire Si/Al supérieur à 8, de préférence, compris entre 10 et 35, et encore plus préférentiellement entre 12 et 35,
- les zéolithes Y en particulier les zéolithes obtenues après traitement de désalumination (par exemple hydrotraitement, lavage à l'aide d'acide chlorhydrique ou traitement par $SiCl_4$) et l'on peut citer plus particulièrement les zéolithes US-Y de rapport molaire Si/Al supérieur à 3, de préférence compris entre 6 et 60 ;
- la zéolithe X de type faujasite de rapport molaire Si/Al de 0,7 à 1,5,
- les zéolithes ZSM-5 ou silicalite d'aluminium de rapport molaire Si/Al de 10 à 500,
- la zéolithe ZSM-11 de rapport molaire Si/Al de 5 à 30.

Parmi toutes ces zéolithes, on fait appel préférentiellement dans le procédé de l'invention aux zéolithes β.

Les zéolithes mises en oeuvre dans le procédé de l'invention, sont des produits connus décrits dans la littérature [cf. Atlas of zeolites structure types by W. M. Meier and D. H. Olson published by the Structure Commission of the International Zeolite Association (1992)].

On peut faire appel aux zéolithes disponibles dans le commerce ou bien les synthétiser selon les procédés décrits dans la littérature.

On peut se référer à l'Atlas précité, et plus particulièrement, pour la préparation :

- de la zéolithe L à la publication de Barrer R. M. et al, Z. Kristallogr., 128, pp. 352 (1969)
- de la zéolithe ZSM-12, au brevet US 3 832 449 et à l'article LaPierre et al, Zeolites 5, pp. 346 (1985),
- de la zéolithe ZSM-22, à la publication Kokotailo G.T. et al, Zeolites 5, pp. 349 (1985),
- de la zéolithe ZSM-23, au brevet US 4 076 842 et à l'article Rohrman A. C. et al, Zeolites 5, pp. 352 (1985),
- de la zéolithe ZSM-48, aux travaux de Schlenker J. L. et al, Zeolites 5, pp. 355 (1985),
- de la zéolithe β, au brevet US 3 308 069 et à l'article Caullet P. et al, Zeolites 12, pp. 240 (1992),
- de la mordénite, aux travaux de Itabashi et al, Zeolites 6, pp. 30 (1986),
- des zéolithes X et Y respectivement aux brevets US 2 882 244 et US 3 130 007,
- de la zéolithe ZSM-5, au brevet US 3 702 886 et à l'article Shiralkar V. P. et al, Zeolites 9, pp. 363 (1989),
- de la zéolithe ZSM-11, aux travaux de Harrison I. D. et al, Zeolites 7, pp. 21 (1987).

La zéolithe constitue la phase catalytique. Elle peut être utilisée seule ou en mélange avec une matrice minérale. Dans la description, on désignera par "catalyseur", le catalyseur réalisé entièrement en zéolithe ou en mélange avec une matrice préparée selon des techniques connues de l'Homme du métier.

A cet effet, la matrice peut être choisie parmi les oxydes de métaux, tels que les oxydes d'aluminium, de silicium et/ou de zirconium, ou encore parmi les argiles et plus particulièrement, le kaolin, le talc ou la montmorillonite.

Dans le catalyseur, la teneur en phase active représente de 5 à 100 % du poids du catalyseur.

Les catalyseurs peuvent se présenter sous différentes formes dans le procédé de l'invention : poudre, produits mis en forme tels que granulés (par exemple, extrudés ou billes), pastilles, qui sont obtenus par extrusion, moulage,

compactage ou tout autre type de procédé connu. En pratique, sur le plan industriel, ce sont les formes de granulés ou de billes qui présentent le plus d'avantages tant sur le plan de l'efficacité que sur le plan de commodité de mise en oeuvre.

Quelle que soit la zéolithe choisie, on fait un traitement si nécessaire qui la rend acide.

A cet effet, on fait appel aux traitements classiques.

Ainsi, on peut échanger les cations alcalins en soumettant la zéolithe à un traitement réalisé avec de l'ammoniaque conduisant ainsi à un échange du cation alcalin par un ion ammonium puis à calciner la zéolithe échangée afin de décomposer thermiquement le cation ammonium et le remplacer par un ion $H^+$.

La quantité d'ammoniaque à mettre en oeuvre est au moins égale à la quantité nécessaire pour échanger tous les cations alcalins en ions $NH_4^+$.

On met donc au moins en jeu de $10^{-5}$ à $5.10^{-3}$ mole d'ammoniaque par gramme de zéolithe.

La réaction d'échange du cation échangeable par $NH_4^+$ est effectuée à une température qui se situe entre la température ambiante et la température de reflux du milieu réactionnel. L'opération dure quelques heures et peut être répétée.

La zéolithe peut être également acidifiée en soumettant celle-ci à un traitement acide classique. Ce traitement peut être effectué par addition d'un acide tel que notamment l'acide chlorhydrique, l'acide sulfurique, l'acide nitrique, l'acide perchlorique, l'acide phosphorique et l'acide trifluorométhanesulfonique.

Selon un mode préférentiel de mise en oeuvre, la zéolithe est acidifiée par passage d'un volume d'acide présentant une normalité comprise entre 0,1 et 2 N par gramme de zéolithe comprise entre 10 ml/g et 100 ml/g. Ce passage peut être réalisé en une seule étape ou de préférence en plusieurs étapes successives.

Conformément à l'invention, la réaction d'acylation est conduite avantageusement en phase liquide comprenant le thioéther aromatique et l'agent d'acylation, en présence du catalyseur.

L'un des réactifs de départ peut servir de solvant réactionnel mais il est également possible de faire appel à un solvant organique.

Comme exemples de solvants convenant à la présente invention, on peut citer en particulier les hydrocarbures aliphatiques ou aromatiques, halogénés ou non, les éther-oxydes aliphatiques, cycloaliphatiques ou aromatiques.

A titre d'exemples d'hydrocarbures aliphatiques, on peut citer plus particulièrement les paraffines tels que notamment, l'hexane, l'heptane, l'octane, le nonane, le décane, le undécane, le dodécane, le tétradécane ou le cyclohexane, et le naphtalène et les hydrocarbures aromatiques et plus particulièrement les hydrocarbures aromatiques comme notamment le benzène, le toluène, les xylènes, le cumène, les coupes pétrolières constituées de mélange d'alkylbenzènes notamment les coupes de type Solvesso®.

En ce qui concerne les hydrocarbures halogénés aliphatiques ou aromatiques, on peut mentionner plus particulièrement, les hydrocarbures perchlorés tels que notamment le tétrachloroéthylène, l'hexachloroéthane ; les hydrocarbures partiellement chlorés tels que le dichlorométhane, le chloroforme, le 1,2-dichloroéthane, le 1,1,1-trichloroéthane, le 1,1,2,2-tétrachloroéthane, le pentachloroéthane, le trichloroéthylène, le 1-chlorobutane, le 1,2-dichlorobutane; le monochlorobenzène, le 1,2-dichlorobenzène, le 1,3-dichlorobenzène, le 1,4-dichlorobenzène, le 1,2,4-trichlorobenzène ou des mélanges de différents chlorobenzènes ; le bromoforme, le bromoéthane ou le 1,2-dibromoéthane ; le monobromobenzène ou des mélanges de monobromobenzène avec un ou plusieurs dibromobenzènes ; le 1-bromonaphtalène.

On peut utiliser également à titre de solvants organiques, les éther-oxydes aliphatiques, cycloaliphatiques ou aromatiques et, plus particulièrement, l'oxyde de diéthyle, l'oxyde de dipropyle, l'oxyde de diisopropyle, l'oxyde de dibutyle, le méthyltertiobutyléther, l'oxyde de dipentyle, l'oxyde de diisopentyle, le diméthyléther de l'éthylèneglycol (ou 1,2-diméthoxyéthane), le diméthyléther du diéthylèneglycol (ou 1,5-diméthoxy 3-oxapentane) ; l'oxyde de benzyle ; le dioxane, le tétrahydrofuranne (THF).

Comme exemples de solvants organiques aprotiques, plus polaires qui peuvent également être mis en oeuvre dans le procédé de l'invention, on peut citer plus particulièrement les composés nitrés comme par exemple, le nitrométhane, le nitroéthane, le 1-nitropropane, le 2-nitropropane ou leurs mélanges, le nitrobenzène ; les nitriles alphatiques ou aromatiques comme l'acétonitrile, le propionitrile, le butanenitrile, l'isobutanenitrile, le benzonitrile, le cyanure de benzyle ; les carboxamides linéaires ou cycliques comme le N,N-diméthylacétamide (DMAC), le N,N-diéthylacétamide, le diméthylformamide (DMF), le diéthylformamide ou la 1-méthyl-2-pyrrolidinone (NMP); le diméthylsulfoxyde (DMSO) ; la tétraméthylènesulfone (sulfolane) ; l'hexaméthylphosphotriamide (HMPT).

Les solvants préférés sont : le dichlorométhane, le tétrachlorométhane, le THF et l'oxyde de diéthyle.

On peut également utiliser un mélange de solvants organiques.

On utilise préférentiellement, le substrat de départ comme solvant réactionnel.

Comme mentionné précédemment, le thioéther aromatique est mis à réagir avec un agent d'acylation, éventuellement en présence d'un solvant réactionnel tel que défini et en présence d'un catalyseur zéolithique.

Le rapport entre le nombre de moles de thioéther aromatique et le nombre de moles d'agent d'acylation peut varier car le substrat peut servir de solvant réactionnel. Ainsi, le rapport peut aller de 0,1 à 10, et se situe de préférence

entre 0,5 et 4,0.

La quantité de catalyseur que l'on met en oeuvre dans le procédé de l'invention peut varier dans de larges limites.

Lorsque l'on réalise le procédé en discontinu, le catalyseur peut représenter en poids par rapport au thioéther aromatique engagé, de 0,01 à 50 %, de préférence, de 5 à 25 %. Cependant si l'on réalise le procédé en continu, par exemple en faisant réagir un mélange du thioéther aromatique et de l'agent d'acylation sur un lit fixe de catalyseur, ces rapports catalyseur/thioéther aromatique n'ont pas de sens et à un instant donné, on peut avoir un excès pondéral de catalyseur par rapport au thioéther aromatique de départ.

Pour ce qui est de la quantité de solvant organique mis en oeuvre, elle est choisie généralement de telle sorte que le rapport entre le nombre de moles de solvant organique et le nombre de moles de thioéther aromatique varie, de préférence, entre 0 et 100 et encore plus préférentiellement entre 0 et 50.

La température à laquelle est mise en oeuvre la réaction d'acylation dépend de la réactivité du substrat de départ et de celle de l'agent d'acylation.

Elle se situe entre 20°C et 300°C, de préférence entre 40°C et 200°C.

Généralement, la réaction est conduite à pression atmosphérique mais des pressions plus faibles ou plus élevées peuvent également convenir. On travaille sous pression autogène lorsque la température de réaction est supérieure à la température d'ébullition des réactifs et/ou des produits.

D'un point de vue pratique, le procédé peut être mis en oeuvre en discontinu ou en continu.

Selon la première variante, il n'y a pas de contraintes au niveau de la mise en oeuvre des réactifs. Ils peuvent être introduits dans un ordre quelconque.

Après mise en contact des réactifs, on porte le mélange réactionnel à la température souhaitée.

L'autre variante de l'invention consiste à conduire la réaction en continu, dans un réacteur tubulaire comportant le catalyseur solide disposé en lit fixe.

Le thioéther aromatique et l'agent d'acylation peuvent être introduits séparément ou en mélange dans le réacteur.

Ils peuvent également être introduits dans un solvant tel que mentionné précédemment.

Le temps de séjour du flux de matière sur le lit catalytique varie, par exemple, entre 15 mn et 10 heures, et de préférence, entre 30 mn et 5 heures.

En fin de réaction, on récupère une phase liquide comprenant le thioéther aromatique acylé qui peut être récupéré de manière classique, par distillation ou par recristallisation dans un solvant approprié, par exemple l'eau ou les alcools (méthanol, éthanol), après élimination préalable des réactifs en excès.

Le procédé de l'invention est particulièrement bien adapté à la préparation de la 4-(méthylthio)acétophénone, par acétylation du thioanisole.

Un avantage du procédé de l'invention est que la réaction d'acylation s'effectue sans qu'il y ait une S-désalkylation du thioéther aromatique de départ.

Les exemples qui suivent, illustrent l'invention sans toutefois la limiter.

Dans les exemples, les rendements mentionnés correspondent à la définition suivante :

$$\text{Rendement : } RR_{A.A.} = \frac{\text{nombre de moles de thioéther aromatique acylé formées}}{\text{nombre de moles d'agent d'acylation introduites}} \%$$

Les exemples qui suivent, illustrent l'invention sans toutefois la limiter.

Exemple 1 :

Dans cet exemple, on met en oeuvre une zéolithe β dont le rapport Si/Al est de 12,5 commercialisée par la Société PQ Zeolites sous la référence CVB 811BL25.

Dans un réacteur fermé de 30 ml, on charge :

- 5 g (40 mmol) de thioanisole commercialisé par Aldrich (pureté = 97 %),
- 2,05 g (20 mmol) d'anhydride acétique,
- 0,5 g de ladite zéolithe β, préalablement calciné à 550°C, sous flux d'air sec.

Le réacteur est chauffé à 90°C pendant 8 heures.

Après 8 heures, le mélange réactionnel est filtré puis analysé par chromatographie en phase vapeur.

On obtient un rendement réactionnel de 60 %.

Exemple 2 :

Dans un réacteur fermé de 30 ml, on charge :

- 35 g (282 mmol) dudit thioanisole,
- 28,7 g (282 mmol) d'anhydride acétique,
- 3,5 g de la zéolithe β décrite dans l'exemple 1, préalablement calciné à 550°C, sous flux d'air sec.

Le réacteur est chauffé à 90°C pendant 8 heures.
Après 12 heures, le mélange réactionnel est filtré puis analysé par chromatographie en phase vapeur.
On obtient un rendement réactionnel de 45 %.

Exemple 3 :

L'exemple suivant est un exemple comparatif.
Dans un réacteur fermé de 30 ml, on charge :

- 2,5 g d'acide acétique,
- 1 ml de thioanisole dans 50 ml de chlorobenzène.

On ajoute ensuite 0,5 g de la zéolithe β décrite dans l'exemple 1,
**[0111]** Après 10 heures à 200°C, on ne détecte pas la formation d'acétothioanisole.

## Revendications

**1.** Procédé d'acylation d'un thioéther aromatique répondant à la formule générale (I) :

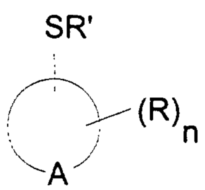

(I)

dans laquelle :

- A symbolise le reste d'un cycle formant tout ou partie d'un système carbocyclique aromatique, monocyclique ou polycyclique, système comprenant au moins un groupe SR' : ledit reste cyclique pouvant porter un ou plusieurs substituants,
- le ou les radicaux R, identiques ou différents, représentent l'un des atomes ou groupes suivants :
  - . un atome d'hydrogène,
  - . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
  - . un radical alcényle linéaire ou ramifié ayant de 2 à 6 atomes de carbone, de préférence, de 2 à 4 atomes de carbone, tel que vinyle, allyle,
  - . un radical cyclohexyle ou benzyle,
  - . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy, isobutoxy, sec-butoxy, tert-butoxy,
  - . un groupe acyle ayant de 2 à 6 atomes de carbone,
  - . un groupe hydroxyle,
  - . un atome d'halogène, de préférence un atome de fluor, chlore ou brome, un radical trifluorométhyle,
  - . un groupe amine,

- R' représente un radical hydrocarboné éventuellement substitué ayant de 1 à 24 atomes de carbone, qui peut être un radical aliphatique acyclique saturé ou insaturé, linéaire ou ramifié ; un radical cycloaliphatique saturé, insaturé ou aromatique, monocyclique ou polycyclique ; un radical aliphatique saturé ou insaturé, linéaire ou ramifié, porteur d'un substituant cyclique,
- R' et R peuvent former un cycle comprenant éventuellement un autre hétéroatome,
- n représentant le nombre de substituants sur un noyau aromatique est un nombre inférieur ou égal à 4,

**caractérisé par le fait qu'**il consiste à faire réagir ledit thioéther avec un agent d'acylation choisi dans le groupe formé par les halogénures d'acides carboxyliques aliphatiques ou cycloaliphatiques et les anhydrides d'acides carboxyliques aliphatiques ou cycloaliphatiques, en présence d'une quantité efficace d'une zéolithe acide.

2. Procédé selon la revendication 1 **caractérisé par le fait que** le thioéther aromatique répond à la formule générale (I) dans laquelle R' représente :

   - un radical aliphatique acyclique, saturé ou insaturé, linéaire ou ramifié, de préférence un radical alkyle linéaire ou ramifié ayant de 1 à 12 atomes de carbone, de préférence de 1 à 6 atomes de carbone : la chaîne hydrocarbonée pouvant être éventuellement interrompue par un hétéroatome, un groupe fonctionnel et/ou porteuse d'un substituant,
   - un radical aliphatique acyclique, saturé ou insaturé, linéaire ou ramifié porteur d'un substituant cyclique éventuellement substitué : ledit radical acyclique pouvant être relié au cycle par un lien valentiel, un hétéroatome ou un groupe fonctionnel,
   - un radical carbocyclique saturé ou comprenant 1 ou 2 insaturations dans le cycle, ayant généralement de 3 à 8 atomes de carbone, de préférence, 6 atomes de carbone dans le cycle ; ledit cycle pouvant être substitué.
   - un radical carbocyclique aromatique, de préférence monocyclique ayant généralement au moins 4 atomes de carbone, de préférence, 6 atomes de carbone dans le cycle ; ledit cycle pouvant être substitué.

3. Procédé selon la revendication 1 **caractérisé par le fait que** le thioéther aromatique répond à la formule générale (I) dans laquelle R' représente un radical alkyle linéaire ou ramifié ayant de 1 à 4 atomes de carbone, de préférence un radical méthyle ou un radical phényle.

4. Procédé selon l'une des revendications 1 à 3 **caractérisé par le fait que** le thioéther aromatique répond à la formule générale (I) dans laquelle le reste A symbolise le reste d'un composé carbocyclique aromatique, monocyclique ayant au moins 4 atomes de carbone et de préférence 6 atomes de carbone ou le reste d'un composé carbocyclique polycyclique qui peut être constitué par au moins 2 carbocycles aromatiques et formant entre eux des systèmes ortho- ou ortho- et péricondensés ou par au moins 2 carbocycles dont au moins l'un d'entre eux est aromatique et formant entre eux des systèmes ortho- ou ortho- et péricondensés : le reste A pouvant porter un ou plusieurs substituants sur le noyau aromatique.

5. Procédé selon l'une des revendications 1 à 4 **caractérisé par le fait que** le thioéther aromatique répond à la formule (Ia) :

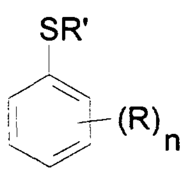

(Ia)

dans laquelle :

   - n est un nombre inférieur ou égal à 4, de préférence égal à 0, 1 ou 2,
   - le radical R' représente un radical alkyle, linéaire ou ramifié, ayant de 1 à 6 atomes de carbone, de préférence de 1 à 4 atomes de carbone, éventuellement interrompu par un atome d'oxygène ou un groupe carbonyle et/ou porteur d'un ou plusieurs atomes d'halogène, de préférence, un atome de chlore, ou un radical phényle,
   - le ou les radicaux R représentent l'un des atomes ou groupes suivants :

     . un atome d'hydrogène,

- . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
- . un radical alcényle linéaire ou ramifié ayant de 2 à 6 atomes de carbone, de préférence, de 2 à 4 atomes de carbone, tel que vinyle, allyle,
- . un radical cyclohexyle ou benzyle,
- . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy, isobutoxy, sec-butoxy, tert-butoxy,
- . un groupe acyle ayant de 2 à 6 atomes de carbone,
- . un groupe hydroxyle,
- . un atome d'halogène, de préférence un atome de fluor, chlore ou brome, un radical trifluorométhyle,
- . un groupe amine,

- deux groupes R placés sur deux atomes de carbone vicinaux peuvent former ensemble et avec les atomes de carbone qui les portent un cycle benzénique,
- les radicaux SR' et R et les 2 atomes successifs du cycle benzénique peuvent former entre eux, un cycle ayant de 5 à 7 atomes, comprenant éventuellement un autre hétéroatome.

**6.** Procédé selon la revendication 5 **caractérisé par le fait que** le thioéther aromatique répond à la formule (Ia) dans laquelle n est supérieur ou égal à 1, les radicaux R' et R et les 2 atomes successifs du cycle benzénique peuvent être liés entre eux par un radical alkylène, alcénylène ou alcénylidène ayant de 2 à 4 atomes de carbone pour former un hétérocycle saturé, insaturé ou aromatique ayant de 5 à 7 atomes de carbone dans lequel un ou plusieurs atomes de carbone peuvent être remplacés par un hétéroatome, de préférence le soufre : les radicaux SR' et R formant de préférence un radical méthylène dithio ou éthylène dithio.

**7.** Procédé selon la revendication 5 **caractérisé par le fait que** le thioéther aromatique répond à la formule (Ia) dans laquelle n est égal à 1, le radical R' représente un radical alkyle ayant de 1 à 4 atomes de carbone et R représente un atome d'hydrogène, un radical alkyle ou alkoxy ayant de 1 à 4 atomes de carbone ou un groupe hydroxyle.

**8.** Procédé selon la revendication 1 **caractérisé par le fait que** le thioéther aromatique est le thioanisole.

**9.** Procédé selon l'une des revendications 1 à 8 **caractérisé par le fait que** l'agent d'acylation répond à la formule (II):

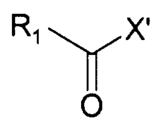

(II)

dans laquelle :

- $R_1$ représente :
  - . un radical aliphatique saturé ou insaturé, linéaire ou ramifié ayant de 1 à 24 atomes de carbone ; un radical cycloaliphatique, saturé ou insaturé, monocyclique ou polycyclique, ayant de 3 à 12 atomes de carbone ;
- X' représente :
  - . un atome d'halogène, de préférence un atome de chlore ou de brome,
  - . un radical $-O-CO-R_2$ avec $R_2$, identique ou différent de $R_1$, ayant la même signification que $R_1$ : $R_1$ et $R_2$ pouvant former ensemble un radical divalent aliphatique saturé ou insaturé, linéaire ou ramifié, ayant au moins 2 atomes de carbone.

**10.** Procédé selon la revendication 9 **caractérisé par le fait que** l'agent d'acylation répond à la formule (II) dans laquelle dans laquelle X' représente un atome de chlore et $R_1$ représente un radical alkyle linéaire ou ramifié ayant de 1 à 12 atomes de carbone, de préférence, 1 à 4 atomes : la chaîne hydrocarbonée pouvant être éventuellement

interrompue par un hétéroatome ou par un groupe fonctionnel ou porteuse de substituants, de préférence, d'atomes d'halogène ; X' représente un radical -O-CO-$R_2$. dans laquelle $R_1$ et $R_2$ sont identiques et représentent un radical alkyle ayant de 1 à 4 atomes de carbone éventuellement porteur d'atomes d'halogène.

**11.** Procédé selon la revendication 9 et 10 **caractérisé par le fait que** l'agent d'acylation est choisi parmi :

- l'anhydride acétique,
- l'anhydride propanoïque,
- l'anhydride isobutyrique,
- l'anhydride trifluoroacétique,
- l'anhydride trichloroacétique,
- l'anhydride de monochloracétyle,
- l'anhydride de dichloroacétyle,
- le chlorure d'acétyle,
- le chlorure de monochloracétyle,
- le chlorure de dichloroacétyle,
- le chlorure de propanoyle,
- le chlorure d'isobutanoyle,
- le chlorure de pivaloyle,
- le chlorure de crotonyle.

**12.** Procédé selon l'une des revendications 1 à 11 **caractérisé par le fait que** la zéolithe est une zéolithe naturelle ou synthétique.

**13.** Procédé selon la revendication 12 **caractérisé par le fait que** la zéolithe est une zéolithe naturelle choisie parmi : la chabazite, la clinoptilolite, l'érionite, la mordénite, la phillipsite, l'offrétite.

**14.** Procédé selon la revendication 13 **caractérisé par le fait que** la zéolithe est une zéolithe synthétique choisie parmi :

- les zéolithes synthétiques à réseau monodimensionnel telles que la zéolithe ZSM-4, la zéolithe L, la zéolithe ZSM-12, la zéolithe ZSM-22, la zéolithe ZSM-23, la zéolithe ZSM-48.
- les zéolithes à réseau bidimensionnel telles que la mordénite, la ferrierite,
- les zéolithes à réseau tridimensionnel telles que la zéolithe β, la zéolithe Y, la zéolithe X, la zéolithe ZSM-5, la zéolithe ZSM-11, l'offrétite.

**15.** Procédé selon la revendication 14 **caractérisé par le fait que** la zéolithe est une zéolithe β de rapport molaire Si/Al supérieur à 8, de préférence, compris entre 10 et 35, et encore plus préférentiellement entre 12 et 35 ou une zéolithe US-Y de rapport molaire Si/Al supérieur à 3, de préférence compris entre 6 et 60.

**16.** Procédé selon l'une des revendications 1 à 15 **caractérisé par le fait que** l'on effectue la réaction en milieu aqueux ou en présence d'un solvant organique choisi parmi les hydrocarbures aliphatiques et/ou aromatiques éventuellement halogénés, de préférence chlorés, les éther-oxydes aliphatiques, cycloaliphatiques ou aromatiques, les solvants aprotiques polaires, de préférence les composés nitrés, les nitriles aliphatiques ou aromatiques, les carboxamides linéaires ou cycliques ; le diméthylsulfoxyde ; la tétraméthylènesulfone ; l'hexaméthylphosphotriamide.

**17.** Procédé selon l'une des revendications 1 à 16 **caractérisé par le fait que** le rapport entre le nombre de moles de thioéther aromatique et le nombre de moles d'agent d'acylation varie entre 0,1 et 10, et se situe de préférence entre 0,5 et 4.

**18.** Procédé selon l'une des revendications 1 à 17 **caractérisé par le fait que** la quantité de catalyseur représente en poids par rapport au thioéther aromatique engagé, de 0,01 à 50 %, de préférence, de 5 à 25 %.

**19.** Procédé selon l'une des revendications 1 à 18 **caractérisé par le fait que** la température à laquelle est mise en oeuvre la réaction d'acylation se situe entre 20°C et 300°C et de préférence, entre 40°C et 200°C.

**20.** Procédé selon l'une des revendications 1 à 19 **caractérisé par le fait qu'**il est conduit en discontinu ou en continu.

## Patentansprüche

1. Verfahren zur Acylierung eines aromatischen Thioethers der allgemeinen Formel (I):

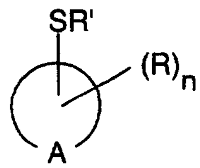

(I)

in der:

- A den Rest eines Ringes, der ganz oder teilweise ein monocyclisches oder polycyclisches, carbocyclisches aromatisches System bildet, wobei das System mindestens eine SR'-Gruppe enthält, darstellt, wobei der cyclische Rest einen oder mehrere Substituenten tragen kann,
- der oder die Reste R, identisch oder verschieden, eines der folgenden Atome oder eine der folgenden Gruppen darstellen:
  - ein Wasserstoffatom,
  - einen linearen oder verzweigten Alkylrest mit 1 bis 6 Kohlenstoffatomen, vorzugsweise mit 1 bis 4 Kohlenstoffatomen, wie Methyl, Ethyl, Propyl, Isopropyl, Butyl, Isobutyl, sec.-Butyl, tert.-Butyl,
  - einen linearen oder verzweigten Alkenylrest mit 2 bis 6 Kohlenstoffatomen, vorzugsweise mit 2 bis 4 Kohlenstoffatomen, wie Vinyl, Allyl,
  - einen Cyclohexyl- oder Benzylrest,
  - einen linearen oder verzweigten Alkoxyrest mit 1 bis 6 Kohlenstoffatomen, vorzugsweise mit 1 bis 4 Kohlenstoffatomen, wie Methoxy, Ethoxy, Propoxy, Isopropoxy, Butoxy, Isobutoxy, sec.-Butoxy, tert.-Butoxy,
  - eine Acylgruppe mit 2 bis 6 Kohlenstoffatomen,
  - eine Hydroxylgruppe,
  - ein Halogenatom, vorzugsweise ein Fluor-, Chlor- oder Bromatom,
  - einen Trifluormethylrest,
  - eine Aminogruppe,

- R' einen gegebenenfalls substituierten Kohlenwasserstoffrest mit 1 bis 24 Kohlenstoffatomen darstellt, der ein linearer oder verzweigter, gesättigter oder ungesättigter, acyclischer aliphatischer Rest; ein monocyclischer oder polycyclischer, gesättigter, ungesättigter oder aromatischer cycloaliphatischer Rest; ein linearer oder verzweigter, gesättigter oder ungesättigter aliphatischer Rest, der einen cyclischen Substituenten trägt, sein kann,
- R' und R einen Ring, der gegebenenfalls ein Heteroatom enthält, bilden können,
- n der Anzahl an Substituenten am aromatischen Kern entspricht und eine Zahl kleiner oder gleich 4 ist,

**dadurch gekennzeichnet, daß** man den Thioether mit einem Acylierungsreagenz, das ausgewählt ist aus der Gruppe von aliphatischen oder cycloaliphatischen Carbonsäurehalogeniden und aliphatischen oder cycloaliphatischen Carbonsäureanhydriden, in Gegenwart einer wirksamen Menge eines sauren Zeoliths reagieren läßt.

2. Verfahren nach Anspruch 1, **dadurch gekennzeichnet, daß** der aromatische Thioether der allgemeinen Formel (I) entspricht, in der R' darstellt:

- einen linearen oder verzweigten, gesättigten oder ungesättigten acyclischen aliphatischen Rest, vorzugsweise einen linearen oder verzweigten Alkylrest mit 1 bis 12 Kohlenstoffatomen, vorzugsweise mit 1 bis 6 Kohlenstoffatomen, wobei die Kohlenwasserstoffkette gegebenenfalls durch ein Heteroatom oder eine funktionelle Gruppe unterbrochen und/oder Träger eines Substituenten sein kann,
- einen linearen oder verzweigten, gesättigten oder ungesättigten, acyclischen aliphatischen Rest, der einen cyclischen, gegebenenfalls substituierten Substituenten trägt, wobei der acyclische Rest mit dem Ring durch eine Valenzbindung, ein Heteroatom oder eine funktionelle Gruppe verbunden sein kann,
- einen gesättigten carbocyclischen Rest mit 1 oder 2 ungesättigten Stellen im Ring, der im allgemeinen 3 bis 8 Kohlenstoffatome, vorzugsweise 6 Kohlenstoffatome, im Ring hat, wobei der Ring substituiert sein kann,

- einem aromatischen carbocyclischen, vorzugsweise monocyclischen Rest mit im allgemeinen mindestens 4 Kohlenstoffatomen, vorzugsweise 6 Kohlenstoffatomen im Ring, wobei der Ring substituiert sein kann.

**3.** Verfahren nach Anspruch 1, **dadurch gekennzeichnet, daß** der aromatische Thioether der allgemeinen Formel (I) entspricht, in der R' einen linearen oder verzweigten Alkylrest mit 1 bis 4 Kohlenstoffatomen, vorzugsweise einen Methylrest oder einen Phenylrest, darstellt.

**4.** Verfahren nach einem der Ansprüche 1 bis 3, **dadurch gekennzeichnet, daß** der aromatische Thioether der allgemeinen Formel (I) entspricht, in der der Rest A dem Rest einer monocyclischen, aromatischen carbocyclischen Verbindung mit mindestens 4 Kohlenstoffatomen und vorzugsweise 6 Kohlenstoffatomen oder dem Rest einer polycyclischen carbocyclischen Verbindung entspricht, die aus mindestens 2 aromatischen Carbocyclen, die untereinander ein ortho- oder ortho- und perikondensiertes System bilden, oder aus mindestens 2 Carbocyclen bestehen kann, von denen mindestens einer aromatisch ist und die untereinander ein ortho- oder ortho- und perikondensiertes System bilden, wobei der Rest A einen oder mehrere Substituenten am aromatischen Kern tragen kann.

**5.** Verfahren nach einem der Ansprüche 1 bis 4, **dadurch gekennzeichnet, daß** der aromatische Thioether der Formel (Ia) entspricht:

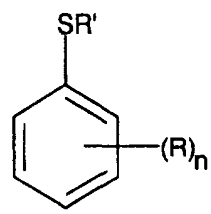

(Ia)

in der:

- n eine Zahl kleiner oder gleich 4, vorzugsweise gleich 0, 1 oder 2, ist,
- der Rest R' einen linearen oder verzweigten Alkylrest mit 1 bis 6 Kohlenstoffatomen, vorzugsweise mit 1 bis 4 Kohlenstoffatomen, der gegebenenfalls durch ein Sauerstoffatom oder eine Carbonylgruppe unterbrochen ist und/oder Träger eines oder mehrerer Halogenatome, vorzugsweise eines Chloratoms, ist, oder einen Phenylrest darstellt,
- der oder die Reste R eines folgenden Atome oder eine der folgenden Gruppen darstellen:

  - ein Wasserstoffatom,
  - einen linearen oder verzweigten Alkylrest mit 1 bis 6 Kohlenstoffatomen, vorzugsweise mit 1 bis 4 Kohlenstoffatomen, wie Methyl, Ethyl, Propyl, Isopropyl, Butyl, Isobutyl, sec.-Butyl, tert.-Butyl,
  - einen linearen oder verzweigten Alkenylrest mit 2 bis 6 Kohlenstoffatomen, vorzugsweise mit 2 bis 4 Kohlenstoffatomen, wie Vinyl, Allyl,
  - einen Cyclohexyl- oder Benzylrest,
  - einen linearen oder verzweigten Alkoxyrest mit 1 bis 6 Kohlenstoffatomen, vorzugsweise mit 1 bis 4 Kohlenstoffatomen, wie Methoxy, Ethoxy, Propoxy, Isoproxy, Butoxy, Isobutoxy, sec.-Butoxy, tert.-Butoxy,
  - eine acyclische Gruppe mit 2 bis 6 Kohlenstoffatomen,
  - eine Hydroxylgruppe,
  - ein Halogenatom, vorzugsweise ein Fluor-, Chlor- oder Bromatom,
  - einen Trifluormethylrest,
  - eine Aminogruppe,

- zwei Gruppen R, die an zwei vicinalen Kohlenstoffatomen befindlich sind, gemeinsam und mit den Kohlenstoffatomen, die sie tragen, einen Benzolring bilden können,
- die Reste SR' und R und die 2 aufeinanderfolgenden Atome des Benzolrings untereinander einen Ring mit 5 bis 7 Atomen, der gegebenenfalls ein weiteres Heteroatom umfaßt, bilden können.

**6.** Verfahren nach Anspruch 5, **dadurch gekennzeichnet, daß** der aromatische Thioether der allgemeinen Formel

(Ia) entspricht, in der n größer oder gleich 1 ist und die Reste R' und R und die 2 aufeinanderfolgenden Atome des Benzolrings untereinander über einen Alkylen-, Alkenylen- oder Alkenylidenrest mit 2 bis 4 Kohlenstoffatomen verbunden sein können, um einen gesättigten, ungesättigten oder aromatischen Heterocyclus mit 5 bis 7 Kohlenstoffatomen zu bilden, in dem ein oder mehrere Kohlenstoffatome gegen ein Heteroatom, vorzugsweise Schwefel, ausgetauscht sein können, wobei die Reste SR' und R vorzugsweise einen Dithiomethylen- oder Dithioethylenrest bilden.

7. Verfahren nach Anspruch 5, **dadurch gekennzeichnet, daß** der aromatische Thioether der allgemeinen Formel (Ia) entspricht, in der n gleich 1 ist, der Rest R' einen Alkylrest mit 1 bis 4 Kohlenstoffatomen darstellt und R ein Wasserstoffatom, einen Alkyl- oder Alkoxyrest mit 1 bis 4 Kohlenstoffatomen oder eine Hydroxylgruppe darstellt.

8. Verfahren nach Anspruch 1, **dadurch gekennzeichnet, daß** der aromatische Thioether Thioanisol ist.

9. Verfahren nach einem der Ansprüche 1 bis 8, **dadurch gekennzeichnet, daß** das Acylierungsreagenz der Formel (II) entspricht:

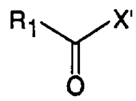

(II)

in der:

- $R_1$ einen linearen oder verzweigten, gesättigten oder ungesättigten aliphatischen Rest mit 1 bis 24 Kohlenstoffatomen; einen monocyclischen oder polycyclischen, gesättigten oder ungesättigten cycloaliphatischen Rest mit 3 bis 12 Kohlenstoffatomen darstellt,
- X' darstellt:
  - ein Halogenatom, vorzugsweise ein Chlor- oder Bromatom,
  - einen Rest $-O-CO-R_2$ mit $R_2$, gleich oder verschieden von $R_1$, mit der gleichen Bedeutung wie $R_1$, wobei $R_1$ und $R_2$ zusammen einen divalenten (zweiwertigen), linearen oder verzweigten, gesättigten oder ungesättigten aliphatischen Rest mit mindestens 2 Kohlenstoffatomen bilden können.

10. Verfahren nach Anspruch 9, **dadurch gekennzeichnet, daß** das Acylierungsreagenz der Formel (II) entspricht, in der X' ein Chloratom darstellt und $R_1$ einen linearen oder verzweigten Alkylrest mit 1 bis 12 Kohlenstoffatomen, vorzugsweise mit 1 bis 4 Kohlenstoffatomen, darstellt, wobei die Kohlenwasserstoffkette gegebenenfalls durch ein Heteroatom oder durch eine funktionelle Gruppe unterbrochen sein kann oder Träger von Substituenten, vorzugsweise von Halogenatomen, sein kann; X' einen Rest $-O-CO-R_2$ darstellt, in dem $R_1$ und $R_2$ identisch sind und einen Alkylrest mit 1 bis 4 Kohlenstoffatomen, der gegebenenfalls Halogenatome trägt, darstellen.

11. Verfahren nach den Ansprüchen 9 und 10, **dadurch gekennzeichnet, daß** das Acylierungsreagenz ausgewählt ist aus:

- Essigsäureanhydrid,
- Propansäureanhydrid,
- Isobutansäureanhydrid,
- Trifluoressigsäureanhydrid,
- Trichloressigsäureanhydrid,
- Monochloressigsäureanhydrid,
- Dichloressigsäureanhydrid,
- Acetylchlorid,
- Monochloracetylchlorid,
- Dichloracetylchlorid,
- Propanylchlorid,
- Isobutylchlorid,
- Pivaloylchlorid,
- Crotonylchlorid.

**12.** Verfahren nach einem der Ansprüche 1 bis 11, **dadurch gekennzeichnet, daß** der Zeolith ein natürlicher oder synthetischer Zeolith ist.

**13.** Verfahren nach Anspruch 12, **dadurch gekennzeichnet, daß** der Zeolith ein natürlicher Zeolith ist, ausgewählt aus der Gruppe von Chabasit, Clinoptilolit, Erionit, Mordenit, Phillipsit und Offretit.

**14.** Verfahren nach Anspruch 13, **dadurch gekennzeichnet, daß** der Zeolith ein synthetischer Zeolith ist, der ausgewählt ist aus der Gruppe von:

- synthetischen Zeolithen mit eindimensionalem Netzwerk wie dem Zeolith ZSM-4, dem Zeolith L, dem Zeolith ZSM-12, dem Zeolith ZSM-22, dem Zeolith ZSM-23, dem Zeolith ZSM-48.
- Zeolithen mit zweidimensionalem Netzwerk wie Mordenit und Ferrierit,
- Zeolithen mit dreidimensionalem Netzwerk wie dem β-Zeolith, dem Zeolith Y, dem Zeolith X, dem Zeolith ZSM-5, dem Zeolith ZSM-11, Offretit.

**15.** Verfahren nach dem Anspruch 14, **dadurch gekennzeichnet, daß** der Zeolith ein β-Zeolith mit einem Si/Al-Molverhältnis größer als 8, vorzugsweise zwischen 10 und 35, besonders bevorzugt zwischen 12 und 35, oder ein Zeolith US-Y mit einem Si/Al-Molverhältnis größer als 3, vorzugsweise zwischen 6 und 60, ist.

**16.** Verfahren nach einem der Ansprüche 1 bis 15, **dadurch gekennzeichnet, daß** man die Reaktion in einer wäßrigen Lösung oder in Gegenwart eines organischen Lösemittels durchführt, das ausgewählt ist aus der Gruppe von gegebenenfalls halogenierten, vorzugsweise chlorierten, aliphatischen und/oder aromatischen Kohlenwasserstoffen, aliphatischen, cycloaliphathischen oder aromatischen Etheroxiden, polaren aprotischen Lösemitteln, vorzugsweise nitrierten Verbindungen, aliphatischen oder aromatischen Nitrilen, linearen oder cyclischen Carboxamiden, Dimethylsulfoxid, Tetramethylensulfon und Hexamethylphosphotriamid.

**17.** Verfahren nach einem der Ansprüche 1 bis 16, **dadurch gekennzeichnet, daß** das Verhältnis zwischen der Anzahl an Mol an aromatischem Thioether und der Anzahl an Mol an Acylierungsreagenz zwischen 0,1 und 10 variiert und vorzugsweise zwischen 0,5 und 4 liegt.

**18.** Verfahren nach einem der Ansprüche 1 bis 17, **dadurch gekennzeichnet, daß** das Gewichtsverhältnis des Katalysators zum eingesetzten aromatischen Thioether 0,01 bis 50 %, vorzugsweise 5 bis 25 %, beträgt.

**19.** Verfahren nach einem der Ansprüche 1 bis 18, **dadurch gekennzeichnet, daß** die Temperatur, bei der die Acylierungsreaktion durchgeführt wird, zwischen 20 °C und 300 °C und vorzugsweise zwischen 40 °C und 200 °C liegt.

**20.** Verfahren nach einem der Ansprüche 1 bis 19, **dadurch gekennzeichnet, daß** das Verfahren diskontinuierlich oder kontinuierlich durchgeführt wird.

## Claims

**1.** A process for the acylation of an aromatic thioether corresponding to general formula (I):

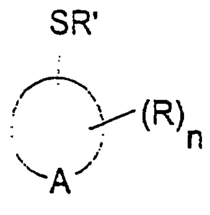

(I)

in which:

- A symbolises the residue of a ring forming all or part of an aromatic, monocyclic or polycyclic carbocyclic system, a system containing at least one SR' group; said cyclic radical may bear one or more substituents,
- the radical(s) R, which may be the same or different, represent one of the following atoms or groups:

- . a hydrogen atom,
- . a linear or branched alkyl radical having 1 to 6 carbon atoms, preferably 1 to 4 carbon atoms, such as methyl, ethyl, propyl, isopropyl, butyl, isobutyl, sec-butyl, tert-butyl,
- . a linear or branched alkenyl radical having 2 to 6 carbon atoms, preferably 2 to 4 carbon atoms, such as vinyl, allyl,
- . a cyclohexyl or benzyl radical,
- . a linear or branched alkoxy radical having 1 to 6 carbon atoms, preferably 1 to 4 carbon atoms, such as the methoxy, ethoxy, propoxy, isopropoxy, butoxy, isobutoxy, sec.-butoxy, tert.-butoxy radicals,
- . an acyl group having 2 to 6 carbon atoms,
- . a hydroxyl group,
- . a halogen atom, preferably a fluorine, chlorine or bromine atom, a trifluoromethyl radical,
- . an amine group,

- R' represents an optionally substituted hydrocarbon radical having 1 to 24 carbon atoms which may be a saturated or unsaturated, linear or branched, acyclic aliphatic radical; a saturated, unsaturated or aromatic, monocyclic or polycyclic, cycloaliphatic radical; a saturated or unsaturated, linear or branched, aliphatic radical bearing a cyclic substituent,
- R' and R may form a ring optionally containing another heteroatom,
- n representing the number of substituents on an aromatic core is a number less than or equal to 4,

**characterised in that** it consists in reacting said thioether with an acylating agent chosen from the group formed by the halides of aliphatic or cycloaliphatic carboxylic acids and the anhydrides of aliphatic or cycloaliphatic carboxylic acids, in the presence of an effective quantity of an acid zeolite.

**2.** A process according to claim 1, **characterised in that** the aromatic thioether corresponds to the general formula (I) in which R' represents:

- a saturated or unsaturated, linear or branched, acyclic aliphatic radical, preferably a linear or branched alkyl radical having 1 to 12 carbon atoms, preferably 1 to 6 carbon atoms; the hydrocarbon chain may optionally be interrupted by a heteroatom, a functional group and/or may bear a substituent,
- a saturated or unsaturated, linear or branched, acyclic aliphatic radical bearing an optionally substituted cyclic substituent; said acyclic radical may be linked to the ring by a valency bond, a heteroatom or a functional group,
- a carbocyclic radical which is saturated or contains 1 or 2 unsaturations in the ring, generally having 3 to 8 carbon atoms, preferably 6 carbon atoms in the ring; said ring may be substituted,
- an aromatic carbocyclic radical, preferably monocyclic, generally having at least 4 carbon atoms, preferably 6 carbon atoms in the ring; said ring may be substituted.

**3.** A process according to claim 1, **characterised in that** the aromatic thioether corresponds to the general formula (I) in which R' represents a linear or branched alkyl radical having 1 to 4 carbon atoms, preferably a methyl radical or a phenyl radical.

**4.** A process according to one of claims 1 to 3, **characterised in that** the aromatic thioether corresponds to the general formula (I) in which the residue A represents the residue of a monocyclic, aromatic carbocyclic compound having at least 4 carbon atoms and preferably 6 carbon atoms or the residue of a polycyclic carbocyclic compound which can be constituted by at least 2 aromatic carbocycles and forming between them ortho- or ortho- and pericondensed systems, or by at least 2 carbocycles, at least one of which is aromatic and forming between them ortho- or ortho- and pericondensed systems: the residue A being able to form one or more substituents on the aromatic core.

**5.** A process according to one of claims 1 to 4, **characterised in that** the aromatic thioether corresponds to the formula (Ia):

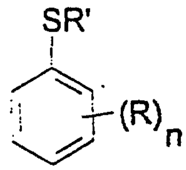

(Ia)

(Ia)

in which:

- n is a number lower than or equal to 4, preferably equal to 0, 1, or 2,
- the radical R' represents a linear or branched alkyl radical having 1 to 6 carbon atoms, preferably 1 to 4 carbon atoms, optionally interrupted by an oxygen atom or a carbonyl group and/or bearing one or more halogen atoms, preferably a chlorine atom, or a phenyl radical,
- the radical(s) R represent one of the following atoms or groups:

  . a hydrogen atom
  . a linear or branched alkyl radical having 1 to 6 carbon atoms, preferably 1 to 4 carbon atoms such as methyl, ethyl, propyl, isopropyl, butyl, isobutyl, sec.-butyl, tert.-butyl,
  . a linear or branched alkenyl radical having 2 to 6 carbon atoms, preferably 2 to 4 carbon atoms, such as vinyl, allyl,
  . a cyclohexyl or benzyl radical,
  . a linear or branched alkoxy radical having 1 to 6 carbon atoms, preferably 1 to 4 carbon atoms such as methoxy, ethoxy, propoxy, isopropoxy, butoxy, isobutoxy, sec.-butoxy, tert.-butoxy radicals,
  . an acyl group having 2 to 6 carbon atoms,
  . a hydroxyl group,
  . a halogen atom, preferably a fluorine, chlorine or bromine atom,
  . a trifluoromethyl radical,
  . an amine group,

- two groups R placed on two vicinal carbon atoms may together, and with the carbon atoms which bear them, form a benzene ring,
- the radicals SR' and R and the 2 successive atoms of the benzene ring may form, amongst themselves, a ring having 5 to 7 atoms, optionally containing another heteroatom.

**6.** A process according to claim 5, **characterised in that** the aromatic thioether corresponds to the formula (Ia) in which n is greater than or equal to 1, the radicals R' and R and the 2 successive atoms of the benzene ring may be linked together by an alkylene, alkenylene or alkenylidene radical having 2 to 4 carbon atoms to form a saturated, unsaturated or aromatic heterocycle having 5 to 7 carbon atoms in which one or more carbon atoms may be replaced by a heteroatom, preferably sulphur; the radicals SR' and R forming preferably a methylenedithio or ethylenedithio radical.

**7.** A process according to Claim 5, **characterised in that** the aromatic thioether corresponds to the formula (Ia) in which n is equal to 1, the radical R' represents an alkyl radical having 1 to 4 carbon atoms and R represents a hydrogen atom, an alkyl or alkoxy radical having 1 to 4 carbon atoms or a hydroxyl group.

**8.** A process according to claim 1, **characterised in that** the aromatic thioether is thioanisole.

**9.** A process according to claim 8, **characterised in that** the acylating agent corresponds to the formula (II):

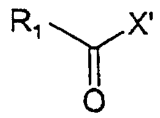

(II)

in which:

- $R_1$ represents:
  . a saturated or unsaturated, linear or branched aliphatic radical having 1 to 24 carbon atoms; a saturated or unsaturated, monocyclic or polycyclic, cycloaliphatic radical having 3 to 12 carbon atoms;
- X' represents:
  . a halogen atom, preferably a chlorine or bromine atom,
  . a radical -O-CO-$R_2$ where $R_2$, which is the same as or different from $R_1$, has the same meaning as $R_1$: R1 and R2 may together form a saturated or unsaturated, linear or branched, aliphatic divalent radical, having at least 2 carbon atoms.

**10.** A process according to claim 9, **characterised in that** the acylating agent corresponds to the formula (II) in which the X' represents a chlorine atom and $R_1$ represents a linear or branched alkyl radical having 1 to 12 carbon atoms, preferably 1 to 4 atoms; the hydrocarbon chain may optionally be interrupted by a heteroatom or by a functional group or may bear substituents, preferably halogen atoms; X' represents an -O-CO-$R_2$ radical in which $R_1$ and $R_2$ are the same and represent an alkyl radical having 1 to 4 carbon atoms optionally bearing halogen atoms.

**11.** A process according to claim 9 and 10, **characterised in that** the acylating agent is chosen from:

- acetic anhydride
- propanoic anhydride
- isobutyric anhydride
- trifluoroacetic anhydride
- trichloroacetic anhydride
- monochloroacetyl anhydride
- dichloroacetyl anhydride
- acetyl chloride
- monochloroacetyl chloride
- dichloroacetyl chloride
- propanoyl chloride
- isobutanoyl chloride
- pivaloyl chloride
- crotonyl chloride.

**12.** A process according to one of claims 11, **characterised in that** the zeolite is a natural or synthetic zeolite.

**13.** A process according to claim 12, **characterised in that** the zeolite is a natural zeolite chosen from: chabazite, clinoptilolite, erionite, mordenite, phillipsite, offretite.

**14.** A process according to claim 13, **characterised in that** the zeolite is a synthetic zeolite chosen from:

. synthetic zeolites with a unidimensional network such as zeolite ZSM-4, zeolite L, zeolite ZSM-12, zeolite ZSM-22, zeolite ZSM-23, zeolite ZSM-48,
. zeolites with a two-dimensional network such as mordenite, ferrierite.
. zeolites with a three-dimensional network such as zeolite-β, zeolite Y, zeolite X, zeolite ZSM-5, zeolite ZSM-11, offretite.

**15.** A process according to claim 14, **characterised in that** the zeolite is a zeolite β with an Si/Al molar ratio greater than 8, preferably between 10 and 35, and even more preferably between 12 and 35, or a zeolite US-Y with an Si/Al molar ratio greater than 3, preferably between 6 and 60.

**16.** A process according to one of claims 1 to 15, **characterised in that** the reaction is carried out in an aqueous medium or in the presence of an organic solvent chosen from optionally halogenated, preferably chlorinated aliphatic and/or aromatic hydrocarbons, aliphatic, cycloaliphatic or aromatic ether oxides, aprotic polar solvents, preferably nitrated compounds, aliphatic or aromatic nitriles, linear or cyclic carboxamides; dimethylsulphoxide; tetramethylenesulphone; hexamethylphosphotriamide.

**17.** A process according to one of claims 1 to 16, **characterised in that** the ratio between the number of moles of aromatic thioether and the number of moles of acylating agent varies between 0.1 and 10, and is preferably between 0.5 and 4.

**18.** A process according to one of claims 1 to 17, **characterised in that** the amount of catalyst represents 0.01 to 50%, preferably 5 to 25%, by weight with respect to the aromatic thioether used.

**19.** A process according to one of claims 1 to 18, **characterised in that** the temperature at which the acylation reaction is brought into effect is between 20°C and 300°C and preferably between 40°C and 200°C.

**20.** A process according to one of claims 1 to 19, **characterised in that** it is carried out discontinuously or continuously.